(19) Europäisches Patentamt
European Patent Office
Office européen des brevets

(11) **EP 3 400 977 B1**

(12) **EUROPÄISCHE PATENTSCHRIFT**

(45) Veröffentlichungstag und Bekanntmachung des Hinweises auf die Patenterteilung:
**17.03.2021 Patentblatt 2021/11**

(51) Int Cl.:
***A61M 1/16*** *(2006.01)*

(21) Anmeldenummer: **18170606.0**

(22) Anmeldetag: **03.05.2018**

(54) **ONLINE LINEARISIERUNG EINES OPTISCHEN SENSORS**

ONLINE LINEARIZATION OF AN OPTICAL SENSOR

LINÉARISATION EN LIGNE D'UN CAPTEUR OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.05.2017 DE 102017110269**

(43) Veröffentlichungstag der Anmeldung:
**14.11.2018 Patentblatt 2018/46**

(73) Patentinhaber: **B. Braun Avitum AG**
**34212 Melsungen (DE)**

(72) Erfinder:
• **JANIK, Waldemar**
**34212 Melsungen (DE)**

• **DURU, Jens**
**36179 Bebra (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Straße 22 85354 Freising (DE)**

(56) Entgegenhaltungen:
EP-A1- 2 783 716          DE-A1-102013 101 523
DE-A1-102013 103 221

EP 3 400 977 B1

## Beschreibung

Die Erfindung betrifft eine Dialysemaschine mit einer dialysatseitigen Abführleitung, einem optischen Sensor in der dialysatseitigen Abführleitung zur Messung des aktuellen Dialyseprozesses und einer Datenkorrektureinrichtung zur Linearisierung des optischen Sensors. Dabei können durch rückwärtiges Extrapolieren der Daten aus dem linearen Bereich die anfänglichen Daten, die im nichtlinearen Bereich aufgenommen wurden, korrigiert werden.

## Hintergrund der Erfindung

Ein Parameter zum Bestimmen der Dialyseeffektivität bzw. der Dialyseeffizienz ist der Kt/V-Wert. Dabei ist K [ml/min] die Clearance (fiktives Plasmavolumen, das pro Zeiteinheit von einer bestimmten Substanz, in anderen Worten eine Konzentration einer Substanz, befreit wird) und wird über den Harnstoffgehalt vor und nach der Dialyse ermittelt. Des Weiteren ist t [min] die effektive Dialysezeit, in anderen Worten die Therapiedauer. V [ml] gibt das Harnstoffverteilungsvolumen an, in anderen Worten 60% der Körpermasse (Gewicht), in der das Blut zirkulieren kann (Körperwassergehalt). Alternativ ist die Clearance K oder das Verhältnis von Clearance zum Verteilungsvolumen K/V auch ein Indikator für die Effektivität einer laufenden Dialysebehandlung.

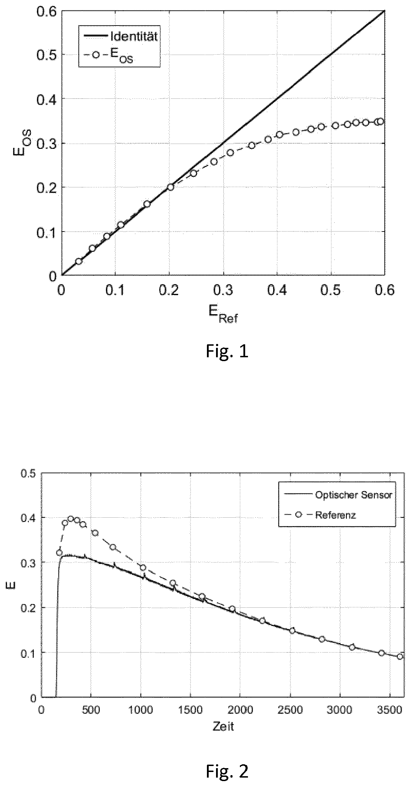

In der Optik ist die Extinktion ein Maß für die Abschwächung einer Strahlung (z.B. sichtbares Licht) nach Durchqueren eines Mediums. Die Extinktion ist wellenlängenabhängig und ist eine logarithmische Größe. Die Extinktion des optischen Sensors Eos aufgetragen gegenüber der Extinktion eines Referenzgerätes $E_{REF}$ (siehe Fig. 1) entspricht bis zu einem gewissen Wert annähernd einer Geraden, danach flacht die Kennlinie ab. Das bedeutet, dass die Kennlinie, die gegen eine Referenz aufgetragen wird, mit zunehmender Konzentration Lichtabsorbierender Stoffe abflacht. In Fig. 1 ist die Extinktion des optischen Sensors $E_{os}$ gegen die Extinktion eines Referenzgerätes $E_{REF}$ aufgetragen, wobei das Referenzgerät einen weiten linearen Bereich aufweist. Zusätzlich ist hier die Identität zu sehen. Demnach ist der optische Sensor bis zu einer Extinktion von ca. 0.2 linear und flacht danach ab.

Im Verlauf einer Dialysetherapie kann ein vorgehend genanntes Verhalten ebenfalls beobachtet werden. Dies gilt insbesondere dann, wenn ein Dialysat viele Lichtabsorbierende Stoffe enthält, was besonders zu Beginn einer Dialyse der Fall ist. Fig. 2 zeigt beispielhaft den zeitlichen Verlauf der Extinktion sowohl für den optischen Sensor, als auch für das Referenzgerät. Dabei sind die beiden Extinktionskennlinien gegenüber der Messdauer (in Sekunden) aufgetragen. Die Differenz zwischen den beiden Extinktionen ist besonders zu Beginn recht hoch. Im weiteren Verlauf nähern sich die Kurven allerdings an, was auf die Abnahme Licht-absorbierender Substanzen zurückzuführen ist. Der optische Sensor befindet sich im linearen Bereich, sobald beide Kennlinien aufeinander liegen. In anderen Worten ausgedrückt, ist besonders zu Beginn das ausgangsseitige Dialysat stark verunreinigt, z.B. durch Harnstoff oder andere urämische Toxine. In diesem Fall ist die Extinktion sehr hoch und der Sensor befindet sich somit im nichtlinearen Bereich, was eine sehr hohe Messunsicherheit zufolge hat. Mit zunehmender Dialysedauer nimmt die Verunreinigung stark ab und der Sensor kommt in den linearen Bereich, in dem die Messwerte wieder mit den Messwerten des Referenzgerätes übereinstimmen.

Im Klinikalltag steht ein Referenzgerät mit weitem linearen Messbereich im Normalfall nicht zur Verfügung. Es kann daher nicht gesagt werden, ob bzw. ab wann sich der optische Sensor in dem linearen Bereich befindet, also ob bzw. ab wann sich der optische Sensor so verhält, wie er sich laut Referenzgerät verhalten sollte.

## Stand der Technik

Das Ermitteln des Kt/V-Wertes wird derzeit durch diverse Onlineverfahren realisiert. Zunächst sei die Methode "Online Clearance Measurement" zu erwähnen. Das Konzept basiert auf dem Prinzip der elektrischen Leitfähigkeitsmessung. Vor und nach dem Dialysator, also am Dialysierflüssigkeitseingang und Dialysierflüssigkeitsausgang, sind zwei Leitfähigkeitssonden installiert. Die Leitfähigkeit in der Dialysierflüssigkeit wird überwiegend durch die Natriumkonzentration bestimmt. Die Messmethode besteht darin, die Leitfähigkeit am Dialysierflüssigkeitseingang kurzzeitig zu erhöhen und den Leitfähigkeitsverlauf am Dialysierflüssigkeitsausgang zu betrachten. Mithilfe der Leitfähigkeit an den Sonden vor und nach dem Dialysator kann dann über den messbaren Unterschied in der Leitfähigkeit die Natrium-Clearance bestimmt werden. Da Natrium Ionen und Harnstoff sehr ähnliche Diffusionscharakteristika aufweisen, kann die Natrium-Clearance direkt in eine Harnstoff- Clearance umgerechnet werden, mit der dann wiederum der Kt/V-Wert ermittelt werden kann. In der Berechnung geht zudem das Verteilungsvolumen V und die Therapiedauer t ein. Bei dieser Methode wird eine Messgenauigkeit von +/- 6% erreicht. Ein Nachteil dieser Methode liegt darin, dass zur kontinuierlichen Überwachung des Kt/V-Wertes in regelmäßigen Abständen die Leitfähigkeit am Dialysierflüssigkeitseingang modifiziert werden muss. Zudem ist die elektrische Leitfähigkeit von Elektrolytlösungen stark abhängig von weiteren gelösten Teilchen, die die Bewegungsfreiheit von Natrium herabsetzen und somit die Leitfähigkeit herabsetzen, was wiederum in falschen Clearance-Berechnungen resultiert. Ein weiterer Nachteil dieser Methode ist, dass Leitfähigkeitsänderungen zu ungewollter Ionen-Aufnahme oder -Entnahme im Patienten führen können. Des Weiteren ist die Leitfähigkeitsänderung träge, was eine kontinuierliche Messung unmöglich macht.

Eine weitere Methode, wie der Kt/V-Wert bestimmt werden kann, besteht darin, einen optischen Sensor am Dialysierflüssigkeitsausgang zu platzieren und Toxine optisch zu detektieren. Das Verfahren nutzt die Eigenschaft aus, dass Harnsäure ein Absorptionsmaximum bei einer Wellenlänge von 290 nm aufweist. Ein optisches System bestehend aus einer UV-LED, die Licht in dieser Wellenlänge emittiert, und einer Photodiode, die eine hohe Empfindlichkeit im UV-Bereich aufweist, kann dazu genutzt werden, die Harnsäurekonzentration im Dialysat qualitativ zu bestimmen. Da die Harnsäure- und die Harnstoffkonzentration im Blut stark korrelieren, kann somit über den optisch ermittelten Abfall der Harnsäurekonzentration während der Therapie auf ein Abfallen der Harnstoffkonzentration und somit auf die Harnstoff-Clearance geschlossen werden. Mittels dieser Clearance kann dann der Kt/V-Wert errechnet werden. Ein gravierender Nachteil dieser Methode besteht darin, dass der verwendete Sensor eine nichtlineare Kennlinie aufweist. Somit wird die Harnstoff-Clearance bei hoher Toxinbelastung im Blut des Patienten und daraus folgender Sättigung des Sensors unterschätzt. Die Nichtlinearität verursacht eine Verfälschung, d.h. Unterschätzung, der Kt/V Berechnung.

Die EP 2 783 716 A1 offenbart ein Verfahren und eine Vorrichtung zur Erkennung einer Rezirkulation in einem Shunt. Dabei gibt ein optischer Sensor am Abfluss eines Dialysators ein Signal, wenn ein vorbestimmter Messwert über- oder unterschritten wird. Basierend auf einer Historie der Therapie oder der Steigung der letzten Messsignale wird ein Sollverlauf berechnet.

Die DE 10 2013 101 523 A1 offenbart eine fotometrische Messvorrichtung und ein Verfahren zum Messen einer optischen Absorbanz eines Fluides mit einer variablen Konzentration von mindestens einer Licht absorbierenden Substanz. Dabei wird das Fluid durch zwei Fluidbehälter geleitet, wobei ein Fluidbehälter schmaler ist als der andere, so dass das Licht zum Messen der Absorbanz unterschiedliche Weglängen in dem Fluid zurücklegen muss.

Die DE 10 2013 103 221 A1 offenbart ein Verfahren zur Erfassung einer Rezirkulation in einem arterievenösen Shunt während eine Hämodialyse. Dabei wird die Rezirkulation anhand des Änderungsverlaufs von einem ersten Parameterwert bei einem ersten Blutflusswert zu einem zweiten Parameterwert bei einem zweiten Blutflusswert ermittelt.

Eine Aufgabe der Erfindung besteht darin, einen bezüglich des Messbereichs nichtlinearen optischen Sensor zumindest während einer laufenden Dialysetherapie zu linearisieren. Auch die nichtinvasive Bestimmung der Clearance und des Verteilungsvolumens ist auf diese Weise möglich. Eine weitere Aufgabe der Erfindung besteht darin, den Verlauf des optischen Sensors auf eine virtuelle Referenz zu projizieren, was mit einer Erweiterung des linearen Messbereichs einhergeht, ohne den Sensor dabei hardwareseitig zu modifizieren oder mit einem Referenzgerät abgleichen zu müssen.

## Kurzbeschreibung der Erfindung

Die Aufgabe wird gelöst durch eine Dialysemaschine bzw. Vorrichtung mit einer Datenkorrektureinrichtung zur Linearisierung des optischen Sensors nach Anspruch 1.

Die Datenkorrektureinrichtung zur Linearisierung eines optischen Sensors in einer Dialysevorrichtung ist dabei vorzugsweise angepasst, dne optischen Sensor durch wiederholte Schaltungen in den Nebenfluss und die folgenden Verfahrensschritte zu linearisieren: das Einbringen eines Sensors in der dialysatseitigen Abführleitung, das Ermitteln des linearen Bereichs des optischen Sensors, sowie das rückwärtige Extrapolieren der Daten aus dem linearen Bereich und das Korrigieren oder das Ersetzen der sensorisch ermittelten Daten aus dem nichtlinearen Bereich, die mittels des optischen Sensors in diesem nichtlinearem Bereich ermittelt worden sind. Mit der Datenkorrektureinrichtung ist eine Erweiterung des Messbereichs, ohne zusätzliche Komponenten möglich, was zu verringerten Kosten führt. Des Weiteren wird die Kt/V-Berechnung optimiert. Auch erlaubt die Erfindung eine Ermittlung blutseitiger Werte, die in der Regel durch sehr hohe Extinktionswerte gekennzeichnet sind.

Das rückwärtige Extrapolieren wird durch nichtlineare Regression einer Regressionskurve durchgeführt. In anderen Worten ausgedrückt wird eine nichtlineare Regression verwendet, um von Werten, die sich im linearen Bereich befinden, auf Werte zu schließen, die sich im nichtlinearen Bereich befinden, wobei der nichtlineare Bereich zu Beginn der Messung gegeben ist und der lineare nach einer zu bestimmenden Messdauer.

Das Ermitteln des linearen Bereichs des optischen Sensors umfasst vorzugsweise das Einstellen eines Nebenschlussintervalls mit einer bestimmten Nebenschlussdauer. Ein Nebenschluss ist gegeben, wenn die Dialysierflüssigkeit durch eine entsprechende Ventilsteuerung am Dialysator vorbeiströmt oder angehalten wird. Die Nebenschlussdauer gibt dabei an, wie lange ein Nebenschluss stattfindet, in anderen Worten über welchen Zeitraum die Dialysierflüssigkeit an dem Dialysator vorbeiströmt , den Dialysator somit überbrückt, oder alternativ angehalten wird. Eine Nebenschlussperiode ist eine zeitliche Periode nachdem sich ein Vorgang, also der Nebenschluss wiederholt. In anderen Worten ist eine Nebenschlussperiode die Zeit von dem Beginn eines Nebenschlusses bis zu dem nächsten Beginn eines weiteren Nebenschlusses. Das Nebenschlussintervall gibt dabei die Zeitdauer von einem Ende eines Nebenschlusses bis zum Beginn des nächsten an. Eine Nebenschlussdauer gibt die Zeit an, wie lange in einen Nebenschluss geschaltet wird.

Das Ermitteln des linearen Bereichs umfasst vorzugsweise durch das Auftragen einer Differenz von lokalen Nebenschlussmaxima und Extinktionssignalen des optischen Sensors 8 unmittelbar vor dem Schalten in den Nebenschluss auf die Extinktionssignale unmittelbar vor dem Schalten in den Nebenschluss und das Ermitteln der Extinktion, die kleiner als ein Hochpunkt ist.

Vorzugweise weisen die Nebenschlüsse eine erste Dauer auf, besonders bevorzugt 18 Sekunden oder weniger, und das Nebenschlussintervall eine zweite Dauer, die länger als die erste Dauer ist, vorzugsweise 4 Minuten. In anderen Worten, ist die Nebenschlussdauer wesentlich kürzer als das Intervall zwischen den Nebenschlüssen. An dieser Stelle sei darauf hingewiesen, dass vorliegend unter "erster Dauer" die Dauer des Nebenschlusses zu verstehen ist und unter der "zweiten Dauer" der zeitliche Abstand zwischen zwei unmittelbar nachfolgenden Nebenschlüssen bzw. Nebenschlussdauern.

Die Nebenschlussintervalle, die Nebenschlussdauern und somit die Nebenschlussperioden können zeitlich äquidistant oder nicht äquidistant verteilt sein. In anderen Worten können die Nebenschlüsse regelmäßig auftreten, was sichere Messergebnisse über den ganzen Messbereich erlaubt oder sie können nicht regelmäßig auftreten, was bei einem erwarteten Wendepunkt zwischen linearem und nichtlinearem Verhalten bei einer größeren Anzahl von Nebenschlüssen, also Messpunkten, in einem kürzeren Zeitraum, eine größere Messgenauigkeit erlaubt.

Die Nebenschlussintervalle oder Nebenschlussperioden können auch erst nach einer vorbestimmten Therapiedauer einsetzen, was den Vorteil hat, dass nur der lineare Bereich gemessen wird und somit die Dauer der Messung verkürzt wird.

Die Clearance K wird vorzugsweise durch eine Berechnung der Extinktion im Plasma bzw. Plasmawasser, vorzugsweise nach einem Nebenschlussmaximum, das auf eine Nebenschlussdauer folgt, die zwischen 2 und 3 Minuten beträgt, berechnet. Im Umkehrschluss bedeutet dies, dass die Bestimmung der dialysatseitigen Clearance und der blutseitigen Clearance erfolgen kann. In anderen Worten kann die blutseitige Clearance nicht-invasiv bestimmt werden.

Vorzugsweise umfassen die für die Linearisierung angepassten Verfahrensschritte ferner das Ermitteln eines Kt/V Wertes, insbesondere anhand einer Regressionskurve. Der Kt/V Wert kann zur Überprüfung des linearisierten optischen Sensors herangezogen werden. Dabei kann das Berechnen des Kt/V Wertes erfolgen durch die Bestimmung des Anfangsharnstoffgehalts c(t) und eine Harnstoffkonzentration zu einem gegebenen Zeitpunkt t, die wiederum durch einen linearisierten optischen Sensor gemessen wurden, durch das Modell zur Berücksichtigung des Rebound Effekts oder durch das Single-Pool-Modell unter Betrachtung der Harnstoffgeneration während der Therapie.

Die Linearisierung des optischen Sensors findet Anwendung in der Dialyse, genauer gesagt in der Dialysemaschine, ist aber nicht nur auf den Bereich einer Dialyse beschränkt. So können die Verfahrensschritte in allen Bereichen verwendet werden, in denen die Konzentration eines absorbierenden Materials nach einem Algorithmus, z.B. einer Exponentialfunktion, sinkt. Die Dialysemaschine mit einem optischen Sensor zur Messung des aktuellen Dialyseprozesses ist gekennzeichnet durch eine Datenkorrektureinrichtung, die dazu angepasst ist, den optischen Sensor nach den vorgehenden Verfahrensschritten zu linearisieren.

**Figurenbeschreibung**

Fig. 1 zeigt die Extinktion eines optischen Sensors gegenüber eines Referenzgerätes,
Fig. 2 zeigt den zeitlichen Verlauf der Extinktion sowohl für den optischen Sensor, als auch für das Referenzgerät,
Fig. 3 zeigt eine schematische Darstellung der Erfindung,
Fig. 4 zeigt einen Extinktionsverlauf während einer Therapie,
Fig. 5 zeigt die Extinktionsdifferenz eines optischen Sensors,
Fig. 6 zeigt die Extinktionsdifferenz eines Referenzmessgeräts,
Fig. 7 zeigt links den dialysatseitigen Extinktionsverlauf der Referenzwerte sowie den exponentiellen Fit der Werte und rechts den dialysatseitigen Extinktionsverlauf des optischen Sensors, sowie extrapolierender, exponentieller Fit der letzten neun Werte,
Fig. 8 zeigt die linearisierte Kennlinie des optischen Sensors, zusammen mit der ursprünglichen Kennlinie,
Fig. 9 zeigt links den blutseitigen Extinktionsverlauf der Referenzwerte sowie den extrapolierten Fit der Werte und rechts den blutseitigen Extinktionsverlauf des optischen Sensors sowie den extrapolierenden, exponentiellen Fit der letzten vier Werte,
Fig. 10 zeigt die linearisierte Kennlinie des optischen Sensors zusammen mit der nichtlinearisierten Kennlinie.

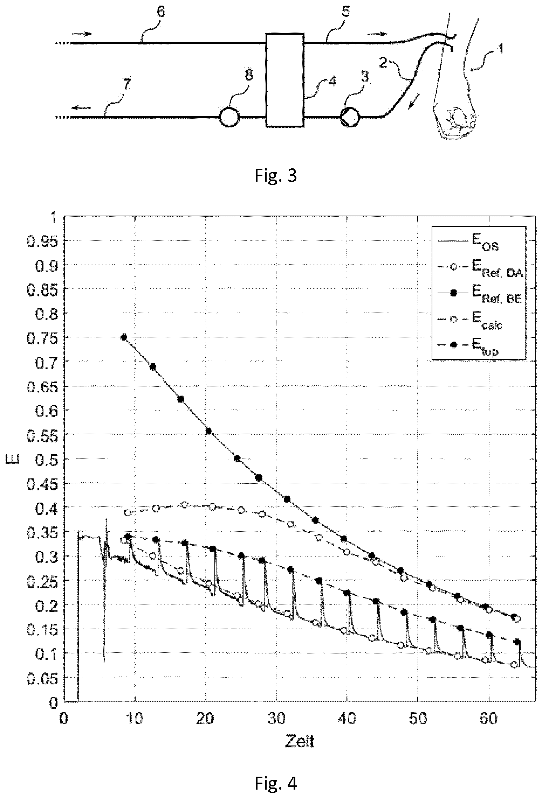

In Fig. 3 ist die schematische Darstellung der Erfindung gezeigt. Einem Patienten 1 wird über das arterielle Schlauchsystem 2, mithilfe einer Fördereinrichtung 3, Blut entnommen, welches zu einem Dialysator gefördert wird. Im Dialysator 4 wird das Blut von harnpflichtigen Substanzen und überschüssigem Wasser befreit. Anschließend wird das gereinigte Blut über das venöse Schlauchsystem 5 dem Patienten zurückgegeben. Die Entnahme und die Rückgabe des Blutes über eine gemeinsame Kanüle ist ebenfalls denkbar. Im Dialysator 4 befinden sich Hohlfaserkapillare, die eine semipermeable Membran aufweisen. Die Kapillare werden von der sogenannten Dialysierflüssigkeit umspült, die zum einen harnpflichtige Stoffe und überschüssiges Wasser aus dem Blut aufnimmt und zum anderen insbesondere Hydrogencarbonat zur Behandlung einer Azidose des Patienten 1 abgibt. Die Dialysierflüssigkeit strömt durch die Zuführleitung 6 zu dem Dialysator. Am Dialysierflüssigkeitsausgang des Dialysators 4 ist eine Abführleitung 7 angebracht, die wenigstens einen optischen Sensor 8 umfasst. Der optische Sensor 8 umfasst zumindest eine Photodiode und vorzugsweise zwei Fotodetektoren und wird zur Bestimmung einer Absorptionseigenschaft eines Dialysats eingesetzt. Dabei handelt es sich bevorzugt, um die Absorbance bzw. Extinktion, die dann gemessen werden kann, wenn das Dialysat Substanzen aufweist, die Licht absorbieren. Die Fotodiode des optischen Sensors 8 emittiert hierzu schmalbandiges Licht im UV-Bereich, wobei die Wellenlängen zwischen 200 und 350 nm bevorzugt werden. Vorzugsweise wird Licht mit einer Peak-Wellenlänge zwischen 275 und 295 nm emittiert. Alternativ ist der optische Sensor so gestaltet, dass er Fluoreszenz misst, wozu er Licht zum Anregen optisch aktiver Substanzen emittiert und anschließend die Emission misst.

Für die Position des optischen Sensors 8 in der Abführleitung 7 ergeben sich unterschiedliche Möglichkeiten. So kann er sich im Falle eines Nebenschlusses im abgetrennten Teil, und/oder vor einer Bilanzierungsvorrichtung oder nach einer Bilanzierungseinrichtung befinden.

Häufig ist die Kennlinie des optischen Sensors 8 nur begrenzt linear (vgl. Fig. 1). Um den linearen Messbereich auszuweiten, wird erfindungsgemäß im Laufe einer Therapie wiederholt in den Nebenschluss geschaltet. Während des Nebenschlusses strömt die Dialysierflüssigkeit durch eine entsprechende Ventilstellung am Dialysator vorbei, wobei das Blut weiterhin durch den Dialysator 4 gefördert wird. Aufgrund des Stoppens (oder alternativ zumindest aufgrund einer Reduzierung) des Dialysierflüssigkeitsflusses durch den Dialysator 4 wird zumindest ein Teil des dialyseseitigen Restvolumens zumindest teilweise gesättigt. D.h., Stoffe, insbesondere lichtabsorbierende Stoffe, auf der Blutseite treten auf die eingeschlossene Dialysatseite über, in anderen Worten ausgedrückt treten in dem Dialysator Stoffe, die mit elektromagnetischer Strahlung wechselwirken, von der Blutführenden Seite auf die Dialysierflüssigkeitführende Seite über. Vorzugsweise sind Nebenschlussdauern von 18 Sekunden vorgesehen. Auch ist der umgekehrte Fall möglich, dass Stoffe von der eingeschlossenen Seite in die Blutseite übertreten.

In Fig. 4 wird ein Extinktionsverlauf während einer Therapie gezeigt. Im Abstand von vier Minuten wird für jeweils 18 Sekunden in den Nebenschluss geschaltet. Das Extinktionssignal des optischen Sensors 8 ($E_{os}$) zeigt nach Beendigung des Nebenschlusses einen kurzzeitigen Anstieg. Die lokalen Maxima werden als $E_{top}$ bezeichnet. $E_{Ref,DA}$ bezeichnet den Verlauf der Extinktion am Dialysierflüssigkeitsausgang, gemessen mit einem Referenzgerät. $E_{Ref,BE}$ bezeichnet den Verlauf der Extinktion am Bluteingang, gemessen mit einem Referenzgerät. $E_{calc}$ bezeichnet den Verlauf der Extinktion am Bluteingang, gemessen mit einem optischen Sensor.

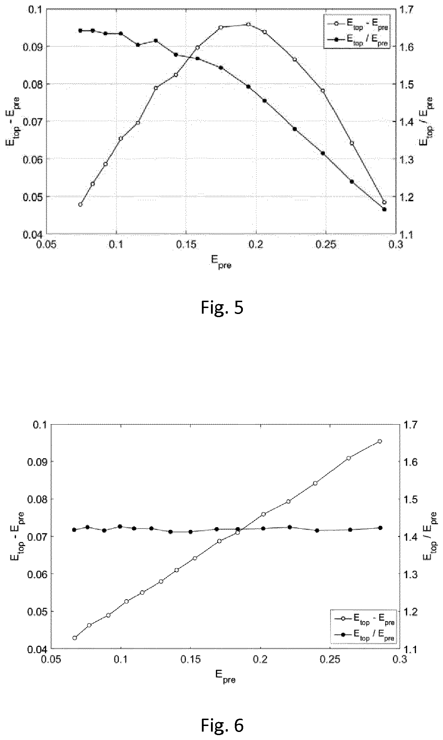

Allein aufgrund des Verlaufs $E_{os}$ ist nicht erkennbar, ob bzw. wann der optische Sensor linear ist. Wird allerdings die Differenz $E_{top}-E_{pre}$ über $E_{pre}$ aufgetragen (wobei $E_{pre}$ für die Extinktion von $E_{os}$ kurz vor dem Schalten in den Nebenschluss steht), so ergibt sich ein charakteristischer Verlauf wie in Fig. 5 dargestellt. Bei einem linearen Sensor wäre die Differenz $E_{top}-E_{pre}$ aufgetragen über $E_{pre}$ eine monoton steigende Gerade (siehe Fig. 6). Im Falle des optischen Sensors weist die Differenz allerdings einen Hochpunkt auf und fällt danach wieder ab. Der $E_{pre}$-Wert im Hochpunkt stellt dabei die Extinktion dar, ab welcher der optische Sensor nichtlinear wird. Extinktionen kleiner als der $E_{pre}$-Wert im Hochpunkt sind somit hinsichtlich Linearität vertrauenswürdig. In anderen Worten ausgedrückt, stammen Extinktionen über dem Hochpunkt aus dem nichtlinearen Bereich und darunter aus dem linearen Bereich. In den Abbildungen ist jeweils auch das Verhältnis $E_{top}/E_{pre}$ gegen $E_{pre}$ dargestellt. Das Verhältnis ist im Falle des Referenzmessgerätes konstant. Im Falle des optischen Sensors gilt eine Konstanz für $E_{pre}$-Werte, die kleiner als die $E_{pre}$-Werte im Maximum sind. Für größer werdende Werte nimmt das Verhältnis kontinuierlich ab. Somit kann anstelle der Differenz auch das Verhältnis herangezogen werden, um beurteilen zu können, ob bzw. ab wann sich der Sensor im linearen Bereich befindet.

Der Verlauf der Differenz in Fig. 5 kann als eine nach unten geöffnete Parabel angesehen werden. Bekanntermaßen ist eine Parabel mathematisch durch drei Punkte definiert. Es ist somit auch denkbar, die Anzahl der Schaltungen in den Nebenschluss auf mindestens drei zu reduzieren, beispielsweise zu Beginn einer Therapie, in der Mitte einer Therapie und am Ende einer Therapie. Über einen anschließenden quadratischen Fit durch diese mindestens drei Punkte kann der Scheitel der Parabel ermittelt werden, der, wie oben beschrieben, als Linearitätsgrenze betrachtet werden kann.

Alternativ sieht es die Erfindung vor, die Durchführung der Nebenschlussschaltungen nur auf den nichtlinearen Bereich, also zu Beginn der Therapie, zu beschränken. Dabei wird in beliebig definierten Zeitabständen in den Nebenschluss geschaltet und anschließend die Differenz betrachtet. Sobald ein Abflachen bzw. die Umkehrung der Steigung der Kurve erkannt wird, kann auf nachfolgende Schaltungen in den Nebenschluss verzichtet werden, da der Sensor ab diesem Punkt linear ist. Der Bereich, in dem das Schalten in den Nebenschluss erfolgen würde, entspräche in Fig. 5 dem rechten Schenkel der Parabel.

Zur Linearisierung werden $E_{pre}$-Werte, die kleiner als die $E_{pre}$-Werte im Maximum sind, herangezogen. Diese Werte werden nun für eine nichtlineare Regression verwendet. Vorzugsweise handelt es sich dabei um eine Exponentialfunktion der Form:

$$E(t) = a \cdot e^{b \cdot t}$$

Aber auch andere Funktionen wie beispielsweise eine doppelte Exponentialfunktion sind denkbar.

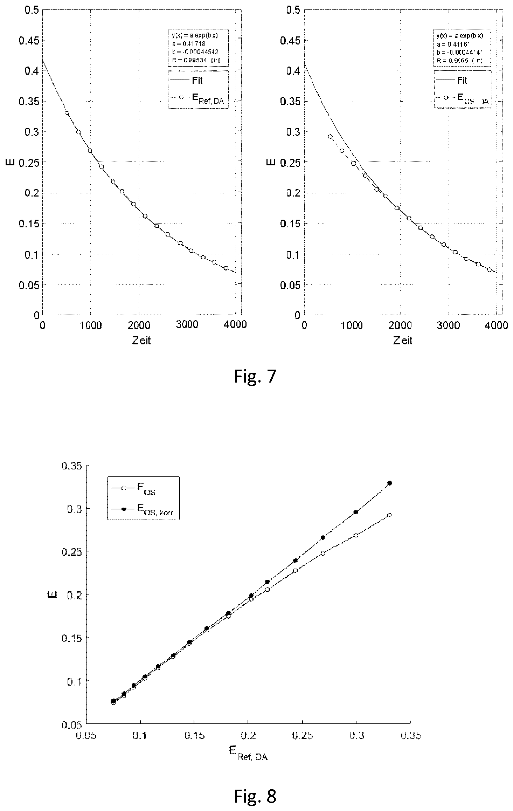

Fig. 7 zeigt zwei Regressionskurven. Links ist die Regression für das Referenzmessgerät dargestellt. Es ist deutlich erkennbar, wie die Messdaten und die Regressionskurve aufeinander liegen. Die extrapolierte Extinktion bei t=0 liegt knapp über 0.4. Rechts ist eine ähnliche Darstellung zu sehen. Allerdings sind hier die Werte des optischen Sensors dargestellt. Es ist zu erkennen, wie hier die gemessenen Extinktionswerte ab einer Extinktion von etwa 0.2 von der Regressionskurve abweichen. Werden die letzten neun Daten gefittet und extrapoliert, so liegt die Extinktion bei t=0 ebenfalls wie die Referenz bei einem Wert von etwas über 0.4. Referenz und Fit des optischen Sensors weisen somit identische Kurvenverläufe auf. Somit wird der optische Sensor 8 während einer Messung online linearisiert.

In Fig. 8 ist nun die gemäß der Erfindung linearisierte Kennlinie des optischen Sensors dargestellt ($E_{OS,korr}$). Zusätzlich ist die ursprüngliche Kennlinie ($E_{os}$) aufgetragen, die es zu korrigieren galt.

Der zeitliche Abstand der Schaltungen in den Nebenschluss ist variabel. Er kann zum Beispiel in fest definierten Abständen während der ganzen Therapiedauer erfolgen, wobei die Zeitpunkte des Schaltens in den Nebenschluss äquidistant oder nicht äquidistant verteilt sein können. Des Weiteren ist denkbar, dass Schaltungen in den Nebenschluss erst nach oder bis zu einer gewissen Therapiedauer einsetzen.

Da die Anfangswerte (Extinktion bei t=0) und die Extinktionswerte zum Ende einer Therapie nun bekannt sind, kann der Kt/V Wert gemäß einer der nachfolgenden Gleichungen korrigiert werden.

Ein vereinfachtes Modell ohne Betrachtung weiterer Effekte ist die einfachste Formel zum Ermitteln des Kt/V-Wertes während einer Dialysetherapie. Diese berücksichtigt weder die Generation von Harnstoff im Patienten während der Therapie, noch den sogenannten Rebound-Effekt.

$$\frac{Kt}{V} = \ln\left(\frac{c_0}{c(t)}\right)$$

Dabei steht K für die Harnstoff-Clearance, t für die Therapiedauer, V für das Harnstoffverteilungsvolumen, co für die Anfangsharnstoffkonzentration und c(t) für die Harnstoffkonzentration zu einem gegebenen Zeitpunkt t.

Ein weiteres Modell für die Bestimmung des Kt/V-Wertes ist das Single-Pool-Modell unter Betrachtung von Harnstoffgeneration während der Therapie. Dabei wird in diesem Modell vereinfacht angenommen, dass Harnstoff lediglich in einem großen Verteilungsvolumen gelöst ist. Im Vergleich zum vorstehenden Modell wird berücksichtigt, dass während der Therapie Harnstoff im Körper des Patienten generiert wird. Des Weiteren berücksichtigt das Modell, dass die durch Ultrafiltration auftretende Konvektion zusätzlich Harnstoff entfernt.

$$sp\frac{Kt}{V} = -\ln\left(-0.008 \cdot t + \frac{c(t)}{c_0}\right) + \left(4 - 3.5 \cdot \frac{c(t)}{c_0}\right) \cdot \frac{UF}{W}$$

Dabei steht UF für das Ultrafiltrationsvolumen und W für das Gewicht des Patienten.

Ein weiteres Modell für die Bestimmung des Kt/V-Wertes berücksichtigt den Rebound-Effekt (equilibrated Kt/V). In der Realität ist die Bewegung von Harnstoff durch den Körper nicht uneingeschränkt möglich, da Harnstoff sowohl im intra- als auch im extrazellulären und im intravasalen Raum vorliegt. Durch ein Modell, das abweichend vom Single-Pool-Modell die Existenz dieser unterschiedlichen Räume berücksichtigt, kann ein sogenannter equilibrated Kt/V ermittelt werden. Das Zurückströmen von Harnstoff nach der Therapie aus Organen mit geringer Blutdurchströmung in den intravasalen Raum wird hierdurch beachtet.

$$e\frac{Kt}{V} = sp\frac{Kt}{V} - \frac{0{,}6}{T} \cdot sp\frac{Kt}{V} + 0{,}03$$

In dieser Formel entspricht T der Gesamttherapiedauer.

Besonders gegen Ende der Dialysetherapie ist die zu erwartende Extinktion niedrig, da viele Licht absorbierende Substanzen bereits entfernt worden sind. Es ist deshalb vorgesehen, insbesondere gegen Ende der Therapie einen langen Nebenschluss von etwa 2 bis 3 Minuten durchzuführen. Während eines Nebenschlusses strömt die Dialysierflüssigkeit am Dialysator vorbei, wobei das Blut weiter zirkuliert. Nach einer gewissen Zeit nimmt das dialysatseitige Restvolumen im Dialysator die Substanzen aus dem Blut in dem Maße auf, dass zwischen der Dialysatseite und der Blutseite im Dialysator ein zumindest teilweise diffusives Gleichgewicht besteht. Wird nun wieder in den Hauptschluss geschaltet, wird das angesättigte dialysatseitige Restvolumen durch den optischen Sensor 8 geleitet, wo eine kurzzeitige Signaländerung gemessen werden kann. Die Extinktion im Maximum der Signaländerung entspricht dabei der Extinktion im Plasma oder zumindest im Plasmawasser. Zur Berechnung der Extinktion im Plasma bzw. im Plasmawasser wird die folgende Gleichung herangezogen:

$$E_{calc} = \left( E_{top} - E_{pre} \right) \cdot k + E_{pre}$$

wobei der Faktor k im Falle eines langen Nebenschlusses 1 ist. Die Clearance K lässt sich bekanntermaßen nach folgender Gleichung ermitteln:

$$K = Q_d \cdot \frac{c_{DO}}{c_{BI}}$$

Dabei steht $Q_d$ für den Dialysierflüssigkeitsfluss und $C_{DO}$ sowie CBI für konzentrationsäquivalente Größen am Dialysierflüssigkeitsausgang und Bluteingang. Eine konzentrationsäquivalente Größe ist beispielsweise eine Konzentration eines oder mehrerer Stoffe oder eine Absorptionseigenschaft wie die Absorbance bzw. Extinktion oder Fluoreszenz. Nach dem Gesetz von Beer-Lambert ist die Extinktion proportional zur Konzentration eines Licht absorbierenden Stoffes. $C_{DO}$ kann direkt mithilfe des optischen Sensors 8 ermittelt werden ($C_{DO}=E_{OS}$). $C_{BI}$ ergibt sich aus den lokalen Maxima, die im Anschluss an einen Nebenschluss auftreten und wie vorstehend berechnet werden ($C_{BI}=E_{calc}$).

Es ist offensichtlich, dass blutseitige Extinktionen immer höher sind als dialysatseitige Extinktionen, sodass das zeitlich begrenzte Schalten in den Nebenschluss bis zum Erreichen eines diffusiven Gleichgewichts immer oder zumindest sehr häufig Sensorsignale in den nichtlinearen Bereich bringen würde. Im schlimmsten Fall würde der optische Sensor sich in der Sättigung befinden, was kaum aussagekräftige Messungen ermöglicht. Labormessungen haben ergeben, dass eine Nebenschlussdauer von 18 Sekunden ausreichend ist, um anschließend 50% des blutseitigen Wertes zu erreichen. Das Risiko, dass der optische Sensor sich in der Sättigung befindet, wird dadurch deutlich verringert. Bezogen auf die Gleichung zur Berechnung der Extinktion im Plasma bedeutet dies, dass k=2 sein muss. Werden andere Dialysatoren oder Flussraten verwendet, sieht die Erfindung vor, den Faktor online zu ermitteln. Hierzu wird zunächst ein langer Nebenschluss und anschließend ein kurzer durchgeführt. Alternativ zuerst ein kurzer und anschließend ein langer. Aus beiden Nebenschlüssen wird zum Schluss das Verhältnis

$$k = \frac{\left( E_{top} - E_{pre} \right)_{lang}}{\left( E_{top} - E_{pre} \right)_{kurz}}$$

gebildet, wobei der Zähler aus dem langen Nebenschluss und der Nenner aus dem kurzen stammt. Wird auf diese Weise der k-Faktor bestimmt, kann anhand kurzer Nebenschlüsse durch dialysatseitige Messungen ein blutseitiger Wert ($E_{calc}$) nichtinvasiv bestimmt werden. Des Weiteren ist es natürlich auch möglich, noch kürzere Nebenschlusszeiten zu wählen, was ebenfalls zu einer Anpassung des k-Faktors führt. Kürzere Nebenschlusszeiten haben den Vorteil, dass die anschließenden lokalen Extinktionsmaxima geringer ausfallen und tendenziell eher im linearen Bereich der Kennlinie des optischen Sensors liegen.

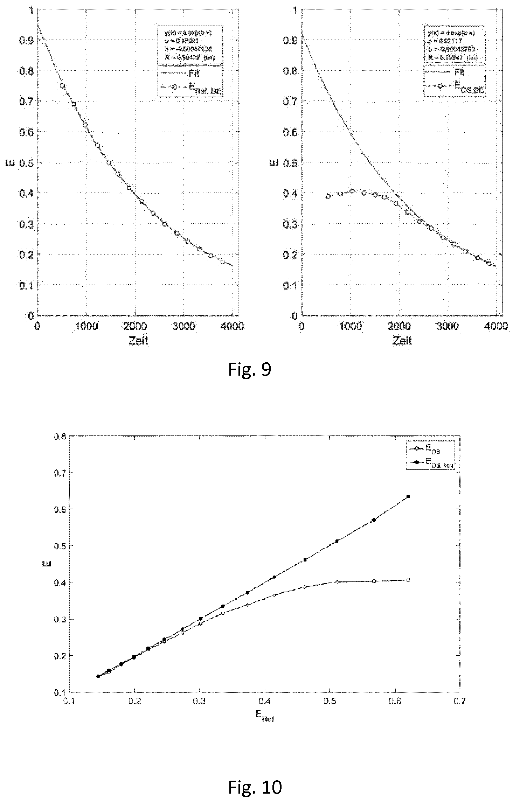

Werden wiederholend kurze Nebenschlüsse und zumindest zum Ende der Therapie mindestens ein langer Nebenschluss durchgeführt, so können durch Bestimmung des k-Faktors und der Gleichung zur Berechnung der Extinktion im Plasma blutseitige Werte ($E_{calc}$) bestimmt werden. Hierzu ist es wichtig, Extinktionen zu verwenden, die im linearen Bereich des optischen Sensors liegen. Das betrifft insbesondere die Extinktionen $E_{top}$ und $E_{pre}$. Welche Werte im linearen Bereich liegen, kann anhand der Abbildung in Fig. 5 entnommen werden. Es lassen sich nun ähnlich wie in den vorherigen Abschnitten nichtlineare Regressionskurven erstellen. In Fig. 9 sind die entsprechenden Regressionen für die Referenz links und den optischen Sensor rechts dargestellt. Im Falle des optischen Sensors wurden die vier letzten berechneten Werte am Bluteingang ($E_{calc}$) herangezogen. Auch in diesem Fall sind die extrapolierten Werte bei t=0 in etwa gleich, wenn die Referenz und optischer Sensor miteinander verglichen werden. Im rechten Teil der Abbildung ist außerdem deutlich zu erkennen, wie die berechneten Werte ($E_{calc}$) zu Beginn der Therapie von der Regressionskurve abweichen. Es ist bekannt, dass die Anzahl der Regressionspunkte großen Einfluss auf die Güte der Extrapolation hat. Um die Extrapolation aus diesem Grund noch robuster bzw. genauer zu gestalten, können zusätzlich zu Beginn und gegen Ende der Therapie Blutproben entnommen und analysiert werden, um anhand der Gleichungen 1, 2 und/oder 3 einen Kt/V-Wert zu bestimmen. Dieser Kt/V-Wert kann dann mit dem Kt/V-Wert aus der Extrapolation verglichen und genutzt werden, um letzteren zu korrigieren.

Fig. 10 zeigt die linearisierte Kennlinie des optischen Sensors zusammen mit der nichtlinearisierten Kennlinie. In der Gleichung zur nichtlinearen Regression steht die Variable b für das Verhältnis von Clearance K zum Verteilungsvolumen V:

$$b = \frac{K}{V}$$

Da b aus Fits (vgl. Fig. 7 und Fig. 9) ermittelt werden kann und K nach Gleichung zur Berechnung der Clearance berechnet wird, kann die Gleichung zur Berechnung der Variablen b nach V umgestellt werden. Auf diese Weise lässt sich das Verteilungsvolumen bestimmen.

Da erstmalig rückwirkend korrigiert wird, kann der Verlauf des optischen Sensors bereits während der Therapie korrigiert werden, also online. Dafür können zum Beispiel die Kennlinien aus Fig. 8 und Fig. 10 herangezogen werden. Außerdem sieht es die Erfindung vor, zumindest die Maxima der Differenz $E_{top}-E_{pre}$ über $E_{pre}$ zu speichern. So kann bei ausreichend verfügbaren Daten für nachkommende Therapien prädiziert werden, ob bzw. ab wann sich die Sensordaten im linearen Bereich befinden. Dadurch können Schaltungen in den Nebenschluss zu Beginn der Therapie vermieden werden.

Alternativ ist eine Ausführungsform ohne zusätzliche Aufnahme von Messwerten denkbar. Dabei ist die Kennlinie des optischen Sensors 8 (oder eines beliebigen anderen Sensors) auf der Maschine und/oder einem Datenmanagementsystem hinterlegt. Diese kann zum Beispiel in Form einer Look-Up-Table realisiert sein und zum Abgleich des optischen Sensors 8 während einer laufenden Therapie genutzt werden. Auch auf diese Weise können Schaltungen in den Nebenschluss vermieden oder zumindest reduziert werden. Da jedes Schalten in den Nebenschluss zur Folge hat, dass das Blut für diesen Zeitraum nicht ausreichend gereinigt werden kann, ist es vorteilhaft, während eines Nebenschlusses weitere Messungen oder Tests durchzuführen, die ebenfalls das Schalten in den Nebenschluss voraussetzen, um die Zeit effizient für multiple Anwendungen zu nutzen.

Bezugszeichenliste

1.   Patient
2.   arterielles Schlauchsystem
3.   Fördereinrichtung
4.   Dialysator
5.   venöses Schlauchsystem
6.   Zuführleitung
7.   Abführleitung
8.   Sensor

**Patentansprüche**

1.   Dialysemaschine mit einer dialysatseitigen Abführleitung und mit einem optischen Sensor (8) in der dialysatseitigen Abführleitung zur Messung des aktuellen Dialyseprozesses und mit einer Datenkorrektureinrichtung, **dadurch gekennzeichnet, dass** die Datenkorrektureinrichtung dazu angepasst ist, den optischen Sensor (8) durch wiederholte Schaltungen in den Nebenschluss und die folgenden Verfahrensschritte zu linearisieren:

Einstellen eines Nebenschlussintervalls und einer bestimmten Nebenschlussdauer,
Ermitteln des linearen Bereichs des optischen Sensors (8),
Rückwärtiges Extrapolieren der anfänglichen Daten im nichtlinearen Bereich durch Daten aus dem linearen Bereich,
Korrigieren oder Ersetzen der sensorisch ermittelten Daten aus dem nichtlinearen Bereich.

2.   Dialysemaschine nach Anspruch 1, **dadurch gekennzeichnet, dass** die Datenkorrektureinrichtung ferner dazu angepasst ist, das rückwärtige Extrapolieren durch nichtlineare Regression einer Regressionskurve durchzuführen.

3.   Dialysemaschine nach Anspruch 2, **dadurch gekennzeichnet, dass** die Datenkorrektureinrichtung ferner dazu angepasst ist, eine Differenz von lokalen Nebenschlussmaxima aufzutragen, vorzugsweise als Minuend und Extinktionssignalen des optischen Sensors (8) unmittelbar vor dem Schalten in den jeweiligen Nebenschluss vorzugsweise als Subtrahend auf die Extinktionssignale unmittelbar vor dem Schalten in den Nebenschluss vorzugsweise als Abszissenachse und das Ermitteln wenigstens einer Extinktion bzw. der Daten, die kleiner als ein Hochpunkt ist/sind.

4. Dialysemaschine nach einem der Ansprüche 2 oder 3, **dadurch gekennzeichnet, dass** die Datenkorrektureinrichtung ferner dazu angepasst ist, die Nebenschlüsse auf eine erste Dauer einzustellen, vorzugsweise 18 Sekunden und/oder weniger, und auf ein Nebenschlussintervall mit einer zweite Dauer einzustellen, die länger als die erste Dauer ist, vorzugsweise 4 Minuten.

5. Dialysemaschine nach einem der Ansprüche 2 bis 4, **dadurch gekennzeichnet, dass** die Datenkorrektureinrichtung ferner dazu angepasst ist, die Nebenschlussintervalle zeitlich äquidistant oder nicht äquidistant zu verteilen.

6. Dialysemaschine nach einem der Ansprüche 2 bis 5, **dadurch gekennzeichnet, dass** die Datenkorrektureinrichtung ferner dazu angepasst ist, eine Clearance K zu bestimmen durch eine Berechnung der Extinktion im Plasma, vorzugsweise nach einem Nebenschlussmaximum, das auf eine Nebenschlussdauer folgt, die zwischen 2 und 3 Minuten beträgt.

7. Dialysemaschine nach Anspruch 6, **dadurch gekennzeichnet, dass** die Datenkorrektureinrichtung ferner dazu angepasst ist, die dialysatseitigen Clearance und die blutseitigen Clearance nicht-invasiv zu bestimmen.

8. Dialysemaschine nach einem der Ansprüche 1 bis 7, **dadurch gekennzeichnet, dass** die Datenkorrektureinrichtung ferner dazu angepasst ist, den Kt/V Wertes zur Überprüfung der Linearisierung des optischen Sensors (8) zu ermitteln.

9. Dialysemaschine nach Anspruch 8, **dadurch gekennzeichnet, dass** die Datenkorrektureinrichtung ferner dazu angepasst ist, den Kt/V-Werte durch die Bestimmung des Anfangsharnstoffgehalt und eine Harnstoffkonzentration c(t) zu einem gegebenen Zeitpunkt zu berechnen, die wiederum durch einen linearisierten optischen Sensor gemessen wurden.

10. Dialysemaschine nach Anspruch 8, **dadurch gekennzeichnet, dass** die Datenkorrektureinrichtung ferner dazu angepasst ist, den Kt/V-Wert durch das Modell zur Berücksichtigung des Rebound Effekts zu ermitteln.

11. Dialysemaschine nach Anspruch 8, **dadurch gekennzeichnet, dass** die Datenkorrektureinrichtung ferner dazu angepasst ist, den ermittelten Kt/V-Wert durch das Single-Pool-Modell unter Betrachtung der Harnstoffgeneration während der Therapie zu ermitteln.

12. Dialysemaschine nach Anspruch 4, **dadurch gekennzeichnet, dass** die Datenkorrektureinrichtung ferner dazu angepasst ist, die Nebenschlussmaxima der Differenz zu speichern.

13. Dialysemaschine nach Anspruch 12, **dadurch gekennzeichnet, dass** die Datenkorrektureinrichtung ferner dazu angepasst ist, die Nebenschlussintervalle nach einer vorbestimmten Therapiedauer einzusetzen.

**Claims**

1. Dialysis machine with a dialysate-side drain line and with an optical sensor (8) in the dialysate-side drain line for measuring the current dialysis process and with a data correction unit, **characterized in that** the data correction unit is adapted to linearize the optical sensor (8) by repeated switching to the shunt and by the following process steps:

   setting a shunt interval and a specific shunt duration,
   determining the linear range of the optical sensor (8),
   backward extrapolating the initial data in the non-linear range by data from the linear range,
   correcting or replacing the sensor-determined data from the non-linear range.

2. Dialysis machine according to claim 1, **characterized in that** the data correction unit is further adapted to perform the backward extrapolation by non-linear regression of a regression curve.

3. Dialysis machine according to claim 2, **characterized in that** the data correction unit is further adapted to apply a difference of local shunt maxima, preferably as minuend and extinction signals of the optical sensor (8) immediately before switching into the respective shunt preferably as subtrahend to the extinction signals immediately before switching into the shunt preferably as abscissa axis and determining at least one extinction or, respectively, the data which is/are smaller than a maximum turning point.

4. Dialysis machine according to one of claims 2 or 3, **characterized in that** the data correction unit is further adapted to set the shunts to a first duration, preferably 18 seconds and/or less, and to set them to a shunt interval having a second duration longer than the first duration, preferably 4 minutes.

5. Dialysis machine according to one of claims 2 to 4, **characterized in that** the data correction unit is further adapted to distribute the shunt intervals equidistantly or non-equidistantly in time.

6. Dialysis machine according to one of claims 2 to 5, **characterized in that** the data correction unit is further adapted to determine a clearance K by a calculation of the extinction in plasma, preferably after a shunt maximum following a shunt duration which is between 2 and 3 minutes.

7. Dialysis machine according to claim 6, **characterized in that** the data correction unit is further adapted to non-invasively determine the dialysate-side clearance and the blood-side clearance.

8. Dialysis machine according to one of claims 1 to 7, **characterized in that** the data correction unit is further adapted to determine the Kt/V value for checking the linearization of the optical sensor (8).

9. Dialysis machine according to claim 8, **characterized in that** the data correction unit is further adapted to calculate the Kt/V value by determining the initial urea content and a urea concentration c(t) at a given point in time, which in turn were measured by a linearized optical sensor.

10. Dialysis machine according to claim 8, **characterized in that** the data correction unit is further adapted to determine the Kt/V value by the model for considering the rebound effect.

11. Dialysis machine according to claim 8, **characterized in that** the data correction unit is further adapted to determine the determined Kt/V value by the Single-Pool Model with consideration of the urea generation during the therapy.

12. Dialysis machine according to claim 4, **characterized in that** the data correction unit is further adapted to store the shunt maxima of the difference.

13. Dialysis machine according to claim 12, **characterized in that** the data correction unit is further adapted to use the shunt intervals after a predetermined duration of therapy.

**Revendications**

1. Machine de dialyse comportant une conduite d'évacuation pour dialysat et un capteur optique (8) dans la conduite d'évacuation pour dialysat pour mesurer le processus de dialyse actuel et un dispositif de correction de données, **caractérisée en ce que** le dispositif de correction de données est adapté pour linéariser le capteur optique (8) par des commutations répétées dans la dérivation et les étapes de procédé suivantes :

   réglage d'un intervalle de dérivation et d'une durée de dérivation déterminée,
   détermination de la plage linéaire du capteur optique (8),
   rétro-extrapolation des données initiales dans la plage non linéaire par des données de la plage linéaire,
   correction ou remplacement des données déterminées par capteur à partir de la plage non linéaire.

2. Machine de dialyse selon la revendication 1, **caractérisée en ce que** le dispositif de correction de données est en outre adapté pour réaliser la rétro-extrapolation par régression non linéaire d'une courbe de régression.

3. Machine de dialyse selon la revendication 2, **caractérisée en ce que** le dispositif de correction de données est en outre adapté pour appliquer une différence de valeurs de dérivation maximales locales, de préférence en tant que montant de soustraction et des signaux d'extinction du capteur optique (8) directement avant la commutation dans la dérivation respective de préférence en tant que terme soustractif sur les signaux d'extinction directement avant la commutation dans la dérivation de préférence en tant qu'axe d'abscisse et la détermination d'au moins une extinction ou des données qui est/sont inférieure(s) à un point culminant.

4. Machine de dialyse selon l'une quelconque des revendications 2 ou 3, **caractérisée en ce que** le dispositif de correction de données est en outre adapté pour régler les dérivations sur une première durée, de préférence de 18 secondes et/ou moins, et sur un intervalle de dérivation avec une seconde durée qui est plus longue que la première durée, de préférence de 4 minutes.

5. Machine de dialyse selon l'une quelconque des revendications 2 à 4, **caractérisée en ce que** le dispositif de correction de données est en outre adapté pour répartir les intervalles de dérivation de façon équidistante dans le temps ou non équidistante.

6. Machine de dialyse selon l'une quelconque des revendications 2 à 5, **caractérisée en ce que** le dispositif de correction de données est en outre adapté pour déterminer une clairance K par un calcul de l'extinction dans le plasma, de préférence selon une valeur maximale de dérivation qui suit une durée de dérivation qui se situe entre 2 et 3 minutes.

7. Machine de dialyse selon la revendication 6, **caractérisée en ce que** le dispositif de correction de données est en outre adapté pour déterminer de façon non invasive la clairance pour le dialysat et la clairance pour le sang.

8. Machine de dialyse selon l'une quelconque des revendications 1 à 7, **caractérisée en ce que** le dispositif de correction de données est en outre adapté pour déterminer la valeur Kt/V pour vérifier la linéarisation du capteur optique (8).

9. Machine de dialyse selon la revendication 8, **caractérisée en ce que** le dispositif de correction de données est en outre adapté pour calculer les valeurs Kt/V en déterminant la teneur initiale en urée et une concentration en urée c(t) à un moment donné qui sont elles-mêmes mesurées par un capteur optique linéarisé.

10. Machine de dialyse selon la revendication 8, **caractérisée en ce que** le dispositif de correction de données est en outre adapté pour déterminer la valeur Kt/V par le modèle de prise en compte de l'effet de rebond.

11. Machine de dialyse selon la revendication 8, **caractérisée en ce que** le dispositif de correction de données est en outre adapté pour déterminer la valeur Kt/V déterminée par le modèle single-pool en prenant en compte la génération d'urée pendant la thérapie.

12. Machine de dialyse selon la revendication 4, **caractérisée en ce que** le dispositif de correction de données est en outre adapté pour enregistrer les valeurs maximales de dérivation de la différence.

13. Machine de dialyse selon la revendication 12, **caractérisée en ce que** le dispositif de correction de données est en outre adapté pour utiliser les intervalles de dérivation selon une durée de thérapie prédéterminée.

Fig. 1

Fig. 2

Fig. 3

Fig. 4

Fig. 5

Fig. 6

Fig. 7

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2783716 A1
- DE 102013101523 A1
- DE 102013103221 A1